# EUROPEAN PATENT APPLICATION

(11) **EP 2 157 421 A1**
(43) Date of publication of application: **24.02.2010**
(21) Application number: 09006141.7
(22) Date of filing: 05.05.2009
(51) Int. Cl.: G01N 21/75, G01N 31/12

(54) **Method for assaying sulfur and apparatus therefor**

(30) Priority: 19.08.2008 JP 2008210912
(71) Applicant: Mitsubishi Chemical Analytech Co., Ltd., Mie-ken (JP)
(72) Inventor: Akasaka, Shuichi, Chigasaki-shi Kanagawa-ken (JP); Tomoyose, Tamaki, Chigasaki-shi Kanagawa-ken (JP)
(74) Representative: HOFFMANN EITLE

(57) **Abstract**

The present invention relates to a sulfur assaying method comprising converting carbon monoxide in a sample gas generated by combustion of the sample to nitrogen dioxide by a pretreatment, and then measuring the intensity of fluorescence of sulfur dioxide in the sample gas, the pretreatment comprising irradiating the sample gas with light from a low pressure mercury lamp, and also relates to a sulfur assaying apparatus comprising a combustor (1) which generates a sample gas from a sample, a pretreatment means (2) for converting nitrogen monoxide in the sample gas to nitrogen dioxide, and an ultraviolet fluorescence detector (4) for measuring the intensity of fluorescence of sulfur dioxide in the sample gas, the pretreatment means (2) comprising a low pressure mercury lamp (3) disposed in a container (20).

## Description

### BACKGROUND OF THE INVENTION

The present invention relates to a method and an apparatus for assaying sulfur, more particularly to a method for assaying sulfur and apparatus therefor which are capable of preventing interference by nitrogen monoxide in carrying out assay of sulfur by an ultraviolet fluorescence method.

Assay of sulfur is conducted, for instance, when rating the quality of Diesel fuels, oils, petroleum products such as gasoline, and the like. Among the known methods for assaying a trace amount of sulfur in a sample, the ultraviolet fluorescence method - a method in which sulfur in a sample is converted to sulfur dioxide to generate a sample gas, then this sample gas is irradiated with ultraviolet light and the intensity of fluorescence of sulfur dioxide is measured - excels in respect of assaying precision.

Regarding the techniques for assaying sulfur according to the above-mentioned ultraviolet fluorescence method, it has been proposed to add ozone to a sample gas to convert nitrogen monoxide contained in the sample gas to nitrogen dioxide as a pretreatment, so as to avoid interference by the absorption wavelength region of nitrogen monoxide in the sample gas to enable precise measurement of peak wavelength of fluorescence by sulfur dioxide in the sample gas (US Patent Application Laid-Open No. 2005/0074365). In a method for ozone incorporation, ozone is generated by an ozone generator (ozonizer) and introduced into a combustor (combustion tube) which generates the specimen gas or supplied to a spot on the downstream side of the combustor

### SUMMARY OF THE INVENTION

The sulfur assays by the methods such as described above involve the problems of increased equipment and management costs and complication of the assaying structure as there is required an ozone forming means such as ozonizer or ozone generator of a high-voltage discharge system or a system in which DC voltage is applied to the electrode elements coated with a solid polymer film.

The present invention has been worked out in view of the above circumstances, and the object thereof is to provide a sulfur assaying method and apparatus for quantitating sulfur in a sample by making use of an ultraviolet fluorescence method, according to which it is possible to prevent interference by nitrogen monoxide in the assays, to simplify the assaying structure and to realize a reduction of cost.

In order to solve the above problem, the present invention incorporates a pretreatment, explained below, in the process for carrying out the analysis of a sample gas formed by combustion of a sample according to an ultraviolet fluorescence method. The pretreatment comprises irradiating the sample gas with light of wavelength of principally 185 nm from a low pressure mercury lamp to generate ozone by making use of oxygen remaining in the sample gas, thereby converting nitrogen monoxide in the sample gas to nitrogen dioxide and suppressing excitation of sulfur dioxide.

In a first aspect of the present invention, there is provided a sulfur assaying method for quantitating sulfur in a sample which contains sulfur and nitrogen, the method comprising the steps of converting sulfur and nitrogen contained in the sample to sulfur dioxide and nitrogen monoxide, respectively, by means of combustion to generate a sample gas, then after converting nitrogen monoxide in the sample gas to nitrogen dioxide by a pretreatment, irradiating the sample gas with ultraviolet light and measuring the intensity of fluorescence of sulfur dioxide, the said pretreatment comprising irradiating the sample gas with light from a low pressure mercury lamp.

In a second aspect of the present invention, there is provided a sulfur assaying apparatus for quantitating sulfur in a sample which contains sulfur and nitrogen, the apparatus comprising a combustor by which sulfur and nitrogen contained in the sample are converted to sulfur dioxide and nitrogen monoxide, respectively, to generate a sample gas, a pretreatment means for converting nitrogen monoxide in the sample gas generated by the combustor to nitrogen dioxide, and an ultraviolet fluorescence detector for measuring the intensity of fluorescence of sulfur dioxide by irradiating the sample gas treated by the said pretreatment means with ultraviolet light, the said pretreatment means comprising a low pressure mercury lamp disposed in a container through which the sample gas is passed.

### BRIEF DESCRIPTION OF THE DRAWING

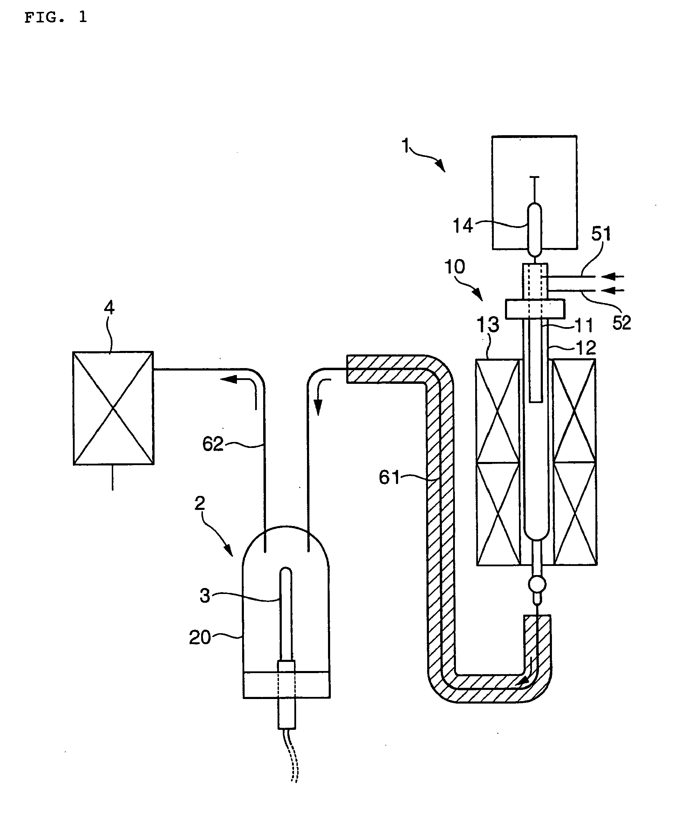

Fig. 1 is a flow chart schematically showing the main structure of the sulfur assaying system according to the present invention.

### DESCRIPTION OF REFERENCE NUMERALS

1: combustor
10: reaction tube
13: heating oven
14: sample injector
2: pretreatment means
20: container
3: low pressure mercury lamp
4: ultraviolet fluorescence detector
51: carrier gas supply channel
52: oxygen supply channel
61: channel
62: channel

### DETAILED DESCRIPTION OF THE INVENTION

An embodiment of the sulfur assaying method and apparatus according to the present invention is explained with reference to the accompanying drawing. Fig. 1 is a flow chart schematically illustrating the main structure of the sulfur assaying system according to the present invention.

First, the sulfur assaying apparatus (hereinafter referred to simply as "assaying apparatus") according to the present invention is explained. The assaying apparatus of the present invention is a system for quantitating sulfur in a sample which contains sulfur and nitrogen by an ultraviolet fluorescence method, and as shown in the accompanying drawing, it comprises principally a combustor (1) serving as a sample gas generating means, a pretreatment means (2) comprising a low pressure mercury lamp (3), and an ultraviolet fluorescence detector (4). With the assaying apparatus of the present invention, it is possible to make assays of not only liquid samples such as Diesel fuels, oils, petroleum products such as gasoline, etc., but also various kinds of gaseous samples by properly selecting means for charging the sample into the combustor (1) to be described later.

Combustor (1) is provided for generating a sample gas from a sample such as mentioned above, and it comprises a reaction tube (10) into which a sample is charged and also oxygen is supplied, and a heating oven (13) for heating the reaction tube (10). The sample in the reaction tube (10) is combusted on heating by the heating oven, whereby sulfur and nitrogen in the sample are converted to sulfur dioxide and nitrogen monoxide, respectively, to generate a sample gas.

The reaction tube (10) has a double-tube structure comprising an inner tube (11) for introducing a sample and an outer tube (12) designed for generating a sample gas. The inner tube (11) is an elongated cylindrical tube which is provided at its top end a sample injector (14) of a syringe injection type by which, for instance, a liquid sample is charged into the system. In the case of a gaseous sample, there is used a device by which the sample is automatically charged into the system by a gas-tight syringe.

The inner tube (11) is designed to be smaller in its outer diameter than the inner diameter of the outer tube (12) and also shorter in its length than the depth of the outer tube (12) so as to provide a space allowing passage of air between the outer peripheral surface of the inner tube (11) and the inner peripheral surface of the outer tube (12). For instance, the inner tube (11) is designed to have a diameter of around 20 to 40 mm and a length of around 100 to 200 mm. Connected to an upper part of the inner tube (11) is a carrier gas supply channel (51) for introducing oxygen for promoting combustion and an inert gas such as argon gas for transfer of the sample.

The outer tube (12) is an elongated bottomed cylindrical tube which is closed at its top end. This tube is designed, for instance, to have a diameter of around 30 to 50 mm and a length of around 300 to 450 mm. Connected to an upper part of the outer tube (12) is an oxygen supply channel (52) for introducing oxygen for combusting the sample. Also connected to the bottom of the outer tube (12) is a channel (61) for letting out the sample gas formed by combustion. The channel (61) comprises a tube of an ion exchange membrane, such as Nafion (registered trademark), for removing moisture in the sample gas.

The heating oven (13) is a means for heating the reaction tube (10). It is usually an electric oven having centrally thereof a hole into which the reaction tube (10) is inserted. The heating oven (13) has a heat insulator applied in its cylindrical casing, with plural heater units being embedded in the heat insulator. The heat insulator is a columnar molding of ceramic fiber or a mixture of ceramic fiber and alumina fiber, and a reaction tube inserting hole is formed along the longitudinal center line thereof.

As the heaters for the heating oven (13), there can be used, for instance, a sheathed heater comprising a Kanthal, Nichrome, silver or like heating element housed in a metallic tube. Such heater units are disposed around the reaction tube inserting hole, with the surface thereof being exposed to the reaction tube inserting hole. There are provided, for instance, about 10 to 12 units of heater, with the total output thereof being set at about 1 kW. In the combustor (1), means are provided for detecting the temperature of the reaction tube (10) and controlling the supply of electricity to the heaters so that the reaction tube (10) will be maintained at a predetermined temperature.

In operation of the combustor (1), a sample is charged into the system by the sample injector (14) and carried into the inner tube (11) and thence into the outer tube (12) by oxygen and an inert gas (argon) supplied from the carrier gas supply channel (51). The outer tube (12) is heated by the heating oven (13) while oxygen is supplied from the oxygen supply channel (52) to let the sample be oxidized and combusted in the outer tube (12). Thereupon sulfur and nitrogen in the sample are converted to sulfur dioxide and nitrogen monoxide, respectively, and the generated sample gas is discharged into and forwarded through the channel (61).

In the present invention, in order to prevent interference by nitrogen when measuring fluorescence of sulfur by an ultraviolet fluorescence method, a pretreatment means (2) for converting nitrogen monoxide in the sample gas formed by the combustor (1) to nitrogen dioxide is provided at a stage rearward of the combustor (1) (on the downstream side thereof in the direction of flow of the collected sample gas). Also, in the present invention, in order to allow easier disposal of nitrogen monoxide, the pretreatment means (2) comprises a low pressure mercury lamp (3) housed in a container (20) where the sample gas is passed.

The low pressure mercury lamp (3) comprises an evacuated glass tube having packed therein vapor of mercury, with its vapor pressure being kept at around 1 Pa, so that the lamp will serve as an electrodischarge light source which radiates the spectral line of mercury alone. This low pressure mercury lamp (3) is preferably one which comprises a quartz glass tube housing a filament. Since such a lamp radiates the bright line of the wavelength of mercury of 185 nm, it makes it possible to efficiently generate ozone from oxygen in the sample gas and convert nitrogen monoxide in the sample gas to nitrogen dioxide. Further, by use of this lamp, it is possible to control (prevent) excitation of the generated sulfur dioxide, eliminating any likelihood of its affecting the ultraviolet fluorescence analysis at a later stage of operation.

A low pressure mercury lamp (3) of the type described above is commercially available, for instance, from Hamamatsu Photonics Ltd. under the trade name of "Pen-type Low Pressure Mercury Lamp L937-1 and L937-2." Such a lamp is characterized by a spark-over voltage of 800 to 900 V, discharge current of 18 mA and discharge maintenance voltage of 200 to 270 V, and it can be lighted by an ordinary low voltage power by use of a power supply system including a transformer.

In the pretreatment means (2), the low pressure mercury lamp (3) is placed, for instance, in a glass-made container (20) after passed through a cover which airtightly closes the open bottom of the container (20). Connected to an appropriate part, for instance, the top end of the container (20) are the channel (61) for introducing a sample gas into the container (20) and a channel (62) for transferring the pretreated sample gas to an ultraviolet fluorescence detector (4). Thus, in the pretreatment means (2), the sample gas introduced into the container (20) through the channel (61) is irradiated with light of mostly 185 nm to generate ozone in the sample gas thereby converting nitrogen monoxide in the sample gas to nitrogen dioxide, while the treated sample gas in the container (20) is transferred into the ultraviolet fluorescence detector (4) through the channel (62).

At a position rearward of the pretreatment means (2) (on the downstream side thereof in the sample gas flowing direction) there is disposed an ultraviolet fluorescence detector (4) in which the pretreated sample gas is irradiated with ultraviolet light and the intensity of fluorescence of sulfur dioxide in the sample gas is measured. This ultraviolet fluorescence detector (4) comprises principally of an ultraviolet lamp which radiates ultraviolet light of a predetermined wavelength and a photomultiplier which receives the ultraviolet fluorescent light.

In the measurement of the intensity of fluorescence by the ultraviolet fluorescence detector (4), the sample gas generated by oxidative combustion is irradiated with ultraviolet light of a wavelength of 190 to 230 nm for sulfur dioxide in the sample gas by using an ultraviolet lamp, and fluorescence of 300 to 450 nm issued accordingly by sulfur dioxide is received by a photomultiplier. That is, the intensity of fluorescence in the process of [SO₂ + h_{ν1} → SO₂ + h_{ν2} (ν1 and ν2: frequencies)] is measured. After conducting waveform processing, the obtained value is expressed as AREA value, and the quantity of sulfur in the sample is determined from the above AREA value by using the calibration curve drawn up in advance with a standard sample.

A sulfur assaying method (hereinafter referred to simply as "assaying method") according to the present invention using the above-described assaying system is explained below. The assaying method of the present invention is a method for quantitating sulfur in a sample which contains sulfur and nitrogen. In the present invention, first oxygen and an inert gas (argon) are supplied as carrier gases into the inner tube (11) of the reaction tube (10) in the combustor (1) through the carrier gas supply channel (51) while oxygen is supplied to the outer tube (12) through the oxygen supply channel (52). Then 10 to 500 µl of a sample (e.g. a fuel oil) is injected into the inner tube (11) by operating the sample injector (14). The pressure and flow rate of the carrier gas and oxygen are set at, for instance, 0.3 to 0.5 MPa and 0.2 to 1.0 L/min, respectively, by adjusting the flow control valves (not shown) provided in the respective supply channels (51) and (52).

For starting injection of the sample, the heating oven (13) is connected to the power to heat the inside of the reaction tube (10) to 600 to 1,100°C. By heating of the reaction tube (10), the sample in the outer tube (12) is combusted to convert sulfur and nitrogen contained in the sample to sulfur dioxide and nitrogen monoxide, respectively, generating a sample gas containing these substances, and this sample gas is let out through the channel (61).

The sample gas generated by the combustor (1) is led into the container (20) of the pretreatment means (2) through the channel (61) and subjected to a pretreatment. In the pretreatment means (2), as described above, the sample gas in the container (20) is irradiated with bright line of a wavelength of 185 nm from a low pressure mercury lamp (3), thereby converting nitrogen monoxide in the sample gas to nitrogen dioxide. According to this pretreatment, ozone can be generated by making use of oxygen remaining in the sample gas, and further, since the sample gas is irradiated with light of a prescribed wavelength, there is no possibility of causing further excitation of sulfur dioxide produced in the sample gas.

The sample gas which has passed through the pretreatment means (2), namely the sample gas containing sulfur dioxide and nitrogen monoxide, is let out through the channel (62) and guided into an ultraviolet fluorescence detector (4) in which, as mentioned above, sulfur dioxide in the sample gas is irradiated with ultraviolet light and the intensity of fluorescence emitted by sulfur dioxide is measured. The quantity of sulfur is calculated from the thus determined intensity of fluorescence by using a separately provided data analyzer such as a computer. Specifically, the quantity of sulfur is calculated based on the calibration curve drawn up in advance with a standard sample, and its result is indicated as the overall sulfur concentration in the sample.

In the present invention, as explained above, in the process for sulfur assaying according to an ultraviolet fluorescence method, there is incorporated a step for a pretreatment of the sample gas, in which the sample gas is irradiated with light from a low pressure mercury lamp (3) by a pretreatment means (2) to generate ozone without causing excitation of sulfur dioxide in the sample gas, and after converting nitrogen monoxide in the sample gas to nitrogen dioxide, sulfur in the sample gas is quantitated by an ultraviolet fluorescence detector (4). Thus, according to the present invention, it is possible to precisely determine the quantity of sulfur in a sample gas by an ultraviolet fluorescence detector (4) without suffering any interference by nitrogen monoxide in the sample. Further, the assaying system can be simplified structurally and the sulfur assaying can be conducted at low cost as compared with the conventional methods using an ozone generator.

According to the present invention, in a pretreatment of a sample gas, the sample gas is irradiated with light of a low pressure mercury lamp to generate ozone without causing excitation of sulfur dioxide in the sample gas, and after converting nitrogen monoxide in the sample gas to nitrogen dioxide, sulfur is quantitated by an ultraviolet fluorescence method, so that the assaying system can be simplified structurally and sulfur assay can be performed at low cost as compared with the conventional methods using an ozone generator.

## Claims

1. A sulfur assaying method for quantitating sulfur in a sample which contains sulfur and nitrogen, the method comprising the steps of converting sulfur and nitrogen contained in the sample to sulfur dioxide and nitrogen monoxide, respectively, by means of combustion to generate a sample gas, then after converting nitrogen monoxide in the sample gas to nitrogen dioxide by a pretreatment, irradiating the sample gas with ultraviolet light and measuring the intensity of fluorescence of sulfur dioxide, the said pretreatment comprising irradiating the sample gas with light from a low pressure mercury lamp.

2. A sulfur assaying apparatus for quantitating sulfur in a sample which contains sulfur and nitrogen, the apparatus comprising a combustor by which sulfur and nitrogen contained in the sample are converted to sulfur dioxide and nitrogen monoxide, respectively, to generate a sample gas, a pretreatment means for converting nitrogen monoxide in the sample gas generated by the combustor to nitrogen dioxide, and an ultraviolet fluorescence detector for measuring the intensity of fluorescence of sulfur dioxide by irradiating the sample gas treated by the said pretreatment means with ultraviolet light, the said pretreatment means comprising a low pressure mercury lamp disposed in a container through which the sample gas is passed.

3. The sulfur assaying apparatus according to claim 2, wherein the low pressure mercury lamp comprises a quartz glass tube housing a filament.

4. The sulfur assaying apparatus according to claim 2 or 3, wherein the combustor comprises a reaction tube into which the sample is charged and also oxygen is supplied, and a heating oven which heats the reaction tube.
